(19) Europäisches Patentamt
European Patent Office
Office européen des brevets

(11) **EP 2 359 281 B1**

(12) **EUROPEAN PATENT SPECIFICATION**

(45) Date of publication and mention
of the grant of the patent:
**24.10.2018 Bulletin 2018/43**

(51) Int Cl.:
***G06F 19/22*** *(2011.01)*      *G06F 19/24* *(2011.01)*
***G06F 19/28*** *(2011.01)*

(21) Application number: **09760322.9**

(22) Date of filing: **11.11.2009**

(86) International application number:
**PCT/IB2009/055000**

(87) International publication number:
**WO 2010/058321 (27.05.2010 Gazette 2010/21)**

(54) **METHOD AND DEVICE FOR EFFICIENT SEARCHING OF DNA SEQUENCE BASED ON ENERGY BANDS OF DNA SPECTROGRAM**

VERFAHREN UND VORRICHTUNG ZUR EFFIZIENTEN SUCHE EINER DNA-SEQUENZ AUF BASIS VON ENERGIEBANDEN EINES DNA-SPEKTROGRAMMS

Procédé et dispositif pour la recherche efficace de séquences d'ADN basée sur les bandes énergétiques du spectrogramme de l'ADN

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.11.2008 EP 08169327**

(43) Date of publication of application:
**24.08.2011 Bulletin 2011/34**

(73) Proprietor: **Koninklijke Philips N.V.**
**5656 AE Eindhoven (NL)**

(72) Inventors:
• **KUDAVELLY, Srinivas, R.**
**NL-5656 AE Eindhoven (NL)**
• **DIMITROVA, Nevenka**
**Eindhoven**
**5656 AE (US)**

(74) Representative: **van Velzen, Maaike Mathilde et al**
**Philips Intellectual Property & Standards**
**High Tech Campus 5**
**5656 AE Eindhoven (NL)**

(56) References cited:
**WO-A-2006/124760      WO-A-2007/105150**

• **AGHILI S A ET AL: "Filtration of string proximity search via transformation" PROCEEDINGS, THIRD IEEE SYMPOSIUM ON BIOINFORMATICS AND BIOENGINEERING (BIBE 2003), PISCATAWAY, NJ, USA, 10 March 2003 (2003-03-10), pages 149-157, XP010637090 ISBN: 978-0-7695-1907-4**
• **TUAN D P ET AL: "LPC-VQ based Hidden Markov Models for Similarity Searching in DNA Sequences" IEEE INTERNATIONAL CONFERENCE ON SYSTEMS, MAN AND CYBERNETICS (ICSMC '06), 1 October 2006 (2006-10-01), pages 1654-1659, XP031117176 ISBN: 978-1-4244-0099-7**
• **NAGARAJAN V ET AL: "A Fourier transformation based method to mine peptide space for antimicrobial activity" BMC BIOINFORMATICS, vol. 7, no. Suppl. 2, September 2006 (2006-09), pages 1-8, XP002511632 ISSN: 1471-2105**
• **MATTEO R ET AL: "Signal Processing in Comparative Genomics" APPLICATIONS OF FUZZY SETS THEORY; [LECTURE NOTES IN COMPUTER SCIENCE], SPRINGER, BERLIN, HEIDELBERG, vol. 4578, 7 July 2007 (2007-07-07), pages 544-550, XP019064613 ISBN: 978-3-540-73399-7**

**Description**

FIELD OF THE INVENTION

This invention pertains in general to the field of DNA sequences analysis. More particularly the invention relates to a method for DNA sequence analysis and a device for DNA sequence analysis.

BACKGROUND OF THE INVENTION

Bioinformatics seeks to organize tremendous volumes of biological data into comprehensible information, which can be used to derive useful knowledge.

One tool commonly used within the field of bioinformatics is the Basic Local Alignment Search Tool (BLAST). To run, BLAST requires a query sequence - also called the target sequence - to search for, and a sequence, or a sequence database containing multiple such sequences, to search against. Based on the query sequence, BLAST will find subsequences in the database which are similar to subsequences in the query. In typical usage, the query sequence is much smaller than the database, e.g., the query may be one thousand nucleotides while the database is several billion nucleotides.

A common problem for BLAST and other search tools known in the art is that the query sequence is limited. If the query sequence length is larger than around a few thousand nucleotides, the search tool will be unacceptably time consuming. Furthermore, with too large query sequences, the accuracy of the search tools diminishes.

In order to make existing bioinformatics tools faster and more accurate, the query sequence is usually manually modified and only the data that is deemed to be most relevant is used for searching. This subjective approch is leading to unreliable results because of unacceptable approximations.

DNA spectral analysis offers an approach to systematically tackle the problem of deriving useful information from DNA sequence data. Generally, DNA spectral analysis involves an identification of the occurrences of each nucleotide base in a DNA sequence as an individual digital signal, and transforming each of the four different nucleotide signals into a frequency domain. The magnitude of a frequency component can then be used to reveal how strongly a nucleotide base pattern is repeated at that frequency. A larger magnitude/value usually indicates a stronger presence of the repetition.

Spectral analysis techniques, such as described in WO 2007/105,150, generally represent an improvement over manual DNA pattern analysis techniques, which aim at identifying DNA patterns serving as biological markers related to important biological processes. Traditionally, automatic analyses are performed directly on strings of DNA sequences composed of the four characters A, T, C and G, which represent the four nucleotide bases. However, due to the tremendous length of DNA sequences (e.g., the length of the shortest human chromosome is 46.9Mb), the wide range of pattern spans associated with the limited character set, and the statistical nature of the problem, such an intuitive/manual approach is inefficient, if not impossible, for achieving the desired purpose.

Hence, an improved method for DNA sequence analysis would be advantageous and in particular a method allowing for increased flexibility, cost-effectiveness, or faster DNA sequence analysis would be advantageous.

SUMMARY OF THE INVENTION

Accordingly, the present invention preferably seeks to mitigate, alleviate or eliminate one or more of the above-identified deficiencies in the art and disadvantages singly or in any combination and solves at least the above mentioned problems e.g. by providing a method for nucleotide sequence analysis based on nucleotide spectrogram database. Such database may e.g. be a DNA database or a RNA database, well known to a person skilled in the art.

In an aspect a method for DNA sequence analysis is provided. The method comprises building a DNA spectrogram database based on a DNA database comprising a number of sequences of nucleotides, by calculating an energy spectral density value for each group of nucleotides comprised in the DNA database. The method further comprises inputting a DNA query sequence. Moreover, the method comprises calculating an energy spectral density value for the DNA query sequence, resulting in an energy spectral density query. The method further comprises calculating a difference between the energy spectral density query value and an energy spectral density value comprised in the DNA spectrogram database. Furthermore, the method comprises selecting a calculated difference, pertaining to a first group of nucleotides, being within a predetermined threshold value range ($\pm \Phi_\Delta$), and performing sequence alignment on said first group of nucleotides from the DNA spectrogram database.

In an aspect a device comprising a processor unit is provided. The processor unit is configured to build a DNA spectrogram database based on a DNA database comprising a number of sequences of nucleotides, by calculating an energy spectral density value for a group of nucleotides comprised in the DNA database. The processor unit is further configured to receive a DNA query sequence. Moreover, ther processor unit is configured to calculate an energy spectral density value for the DNA query sequence, resulting in an energy spectral density query. Furthermore, the processor unit is configured to calculate a difference between the energy spectral density query value and an energy spectral density value comprised in the DNA spectrogram database. The processor unit is further configured to select a difference being lower than a predetermined threshold value; and to perform sequence alignment of the nucleotides comprised in a selected group.

In yet another aspect a computer-readable medium having embodied thereon a computer program for processing by a processor is provided. The computer program comprises a first code segment for building a DNA spectrogram database based on a DNA database comprising a number of sequences of nucleotides, by calculating an energy spectral density value for a group of nucleotides comprised in the DNA database. The computer program further comprises a second code segment for inputting a DNA query sequence. Moreover, the computer program comprises a third code segment for calculating an energy spectral density value for the DNA query sequence, resulting in an energy spectral density query. Furthermore, the computer program comprises a fourth code segment for calculating a difference between the energy spectral density query value and an energy spectral density value comprised in the DNA spectrogram database. The computer program also comprises a fifth code segment for selecting a difference being lower than a predetermined threshold value; and a sixth code segment for performing sequence alignment of the nucleotides comprised in a selected group.

The method comprises the steps of building a DNA spectrogram database. The spectrogram database is based on a DNA database comprising a number of sequences of nucleotides. This is done by calculating an energy spectral density value for a group of nucleotides comprised in the DNA database. A DNA query sequence is used as an input. The energy spectral density value for the DNA query sequence is calculated, resulting in an energy spectral density query. Then, a difference between the energy spectral density query value and an energy spectral density value comprised in the DNA spectrogram database is calculated. After this, a calculated difference, pertaining to a first group of nucleotides, being within a predetermined threshold value range ($\pm \Phi_\Delta$) is selected, and sequence alignment on said first group of nucleotides from the DNA spectrogram database is performed.

The present invention according to some embodiments has the advantage over the prior art that it provides a possibility to compare sequences with large number of nucleotides. Moreover, the improved sequence comparison may also be performed faster than current solutions.

Other embodiments of the invention will be explained in further detail below.

## BRIEF DESCRIPTION OF THE DRAWINGS

These and other aspects, features and advantages of which the invention is capable of will be apparent and elucidated from the following description of embodiments of the present invention, reference being made to the accompanying drawings, in which

Fig. 1    is a flowchart of a method according to an embodiment;
Fig. 2    is a flowchart of the building step of the method according to an embodiment; and
Fig. 3    is a block diagram of a device according to according to an embodiment.
Fig. 4    is a block diagram of a computer-readable medium according to an embodiment.

## DETAILED DESCRIPTION OF EMBODIMENTS

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

The following description focuses on embodiments of the present invention applicable to efficient searching of DNA Sequence in a DNA sequence database based on energy bands of DNA Spectrogram.

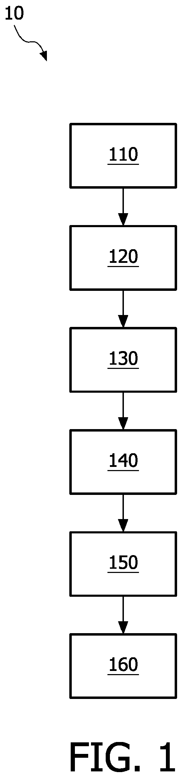

In an embodiment, according to Fig. 1, a method 10 for DNA sequence analysis is disclosed. The method comprises building 110 a DNA spectrogram database based on a DNA database comprising a number of sequences of nucleotides, by calculating an energy spectral density value for a group of nucleotides comprised in the DNA database. The method may further comprise inputting 120 a DNA query sequence. Moreover, the method comprises calculating 130 an energy spectral density value for the DNA query sequence, resulting in an energy spectral density query. Furthermore, the method may comprise calculating a difference 140 between the energy spectral density query value and an energy spectral density value comprised in the DNA spectrogram database. The method may also comprise selecting 150 a difference being lower than a predetermined threshold value.

The group of nucleotides, corresponding to the selected difference, may then be further processed using sequence alignment e.g. a BLAST algorithm. Accordingly, the method may further comprise performing 160 sequence alignment the nucleotides comprised in a selected group.

According to one embodiment, the DNA spectrogram database is an energy spectral density (ESD) database. The DNA spectrogram database may be a genomic DNA spectral database. The ESD describes how the energy (or variance) of a signal or a time series is distributed with frequency. If f(t) is a finite-energy (square integrable) signal, the spectral density $\Phi(\omega)$ of the signal is the square of the magnitude of the continuous Fourier transform of the signal. The energy is represented by the integral of the square of a signal.

As the signal is discrete with values $f_n$, over an infinite number of elements, we still have an energy spectral density:

$$\Phi(\omega) = \left| \frac{1}{\sqrt{2\pi}} \sum_{n=-\infty}^{\infty} f_n e^{-j\omega n} \right|^2 = \frac{F(\omega)F^*(\omega)}{2\pi}$$

where $\omega$ is the angular frequency ($2\pi$ times the cycle frequency) and $F(\omega)$ is the discrete-time Fourier transform of $f_n$, and $F^*(\omega)$ is its complex conjugate. The multiplicative factor of $1/2\pi$ is not absolute, but rather depends on the particular normalizing constants used in the definition of the various Fourier transforms.

According to one embodiment a set of color spectrums of the nucleotide segment, such as a DNA segment, is achived in a way well known to a person skilled in the art. Next, the periodicity of different color spectrums is calculated by the formula:

$$Periodicity = \frac{STFT\,Window\,Size}{Frequency}$$

Here, STFT Window Size is the window size calculated by Short Time Fourier Transform (STFT), well known to a person skilled in the art, and Frequency is the frequency of which a certain color spectrum is occuring when the different color spectrums are aligned. For a particluar STFT Window Size, Discrete Fourier Transforms (DFT) are combined in the color space, indicating a certain frequency. Then, the DFT values are squared and divided with the STFT Window Size to get the ESD.

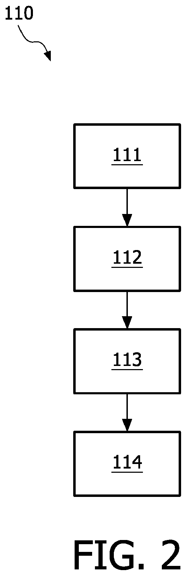

In an embodiment according to Fig. 2, the building 110 of a DNA spectrogram database is shown. First DNA spectrograms are pre-computed 111 for a large number of genome sequences. A large number of ESD are computed according to above for various lengths of sequences, comprised in a DNA sequence database, and various overlapping starting points. Such pre-computed ESD values may be used as part of the header information of the query sequence similar to a FASTA header, known in the art. The ESD values may differ for a range of nucleotide lengths, e.g. $\Phi_1$, $\Phi_2$,..., $\Phi_n$ for nucleotide lengths 256, 1024 ... , 8196 respectively. This may trigger the query and make another computation of ESD unnecessary. For example, in a certain color space, ESD computation may be derived by squaring DFT values and dividing them by the STFT Window Size.

The building 110 of the DNA spectrogram database may further comprise indexing 112 the pre-computed 111 DNA spectrograms in a structure based on phylogenetic distances. The building 110 of the DNA spectrogram database may further comprise assigning 113 a pointer to the spectrograms. Such pointer may be e.g. a reference to a local database, a URL to a web resource or a protected sequence. The spectrograms may then be stored 114.

In an embodiment, an ESD database may be used in such a way as to provide a fast baseline of probable candidates of sequences from the DNA sequence database, wherein the candidates may be related to the query sequence based on the ESD. Accordingly, the candidates having a similar ESD value to the ESD value of the query sequence may rapidly be identified for further processing. This is due to the fact that the method identifies sequences having similar ESD values to the ESD value of the query sequence. Accordingly, sequences having ESD values within $\pm \Phi_\Delta$, may be selected for subsequent processing.

The ESD database also gives the possibility to identify mutations in the DNA sequence. If the specific DNA sequence location e.g. already is known, the energy spectral density ($\Phi_{\text{Re } f}$) of the "healthy / valid" sequence is computed. In order to check for any mutation at that location in other DNA sequences, instead of comparing the sequence per nucleotide, in accordance with current solutions, the "energy spectral density" may be computed directly and changes in value of the "energy spectral density ($\Phi_{sam}$)" may be checked for . If $\Phi_{\text{Re } f} \neq \Phi_{sam}$, then there is a mutation, and whether it is fatal or not needs to be compared in depth using the existing search tools like BLAST.

In another embodiment the method comprises comparing "entire" chromosome or genomic sequence against the database of stored sequences without any huge penalty of comparing every nucleotide for producing search results, as the comparison is based on the "energy spectral density".

According to one embodiment, the sequence alignment 160 is local alignment, such as alignment of short sequences or alignment of shot-gun sequencing results.

According to another embodiment, the sequence alignment 160 is global alignment, such as alignment of multiple sequences all at once or alignment of two or more genomes.

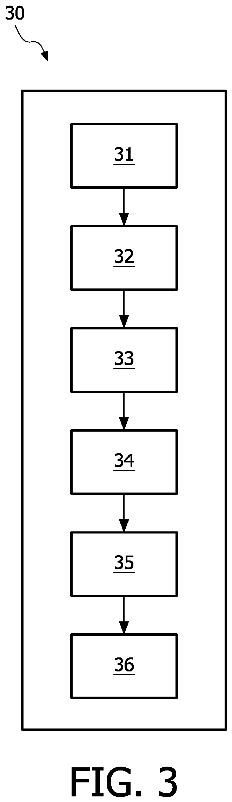

In an embodiment, according to Fig. 3, a device 30 is provided. The device comprises a processor unit configured to build 31 a DNA spectrogram database based on a DNA database comprising a number of sequences of nucleotides, by calculating an energy spectral density value for a group of nucleotides comprised in the DNA database. The processor unit is further configured to receive 32 a DNA query sequence. Moreover, the processor is configured to calculate 33 an energy spectral density value for the DNA query sequence, resulting in an energy spectral density query. Furthermore, the processor unit is configured to calculate 34 a difference 140 between the energy spectral density query value and an energy spectral density value comprised in the DNA spectrogram database. The processor unit is further configured to select 35 a difference being lower than a predetermined threshold value.

The processor unit is further configured to perform 36 sequence alignment the nucleotides comprised in a selected group.

In an embodiment the processor unit is configured to perform any one of the steps of the method according to some embodiments.

According to another embodiment, any of the abovementioned method may be used for designing test kits for diagnosing genetic diseases.

In one embodiment, a clinical genetics program is disclosed, the program comprising means to provide fast access to similar genomes of patients with similar disease conditions or provide fast access to similar patients with similar therapy response. The program may also comprise information from pharmacological databases for therapy response and associated genes with this therapy response as well as storage of genomic sequencing (like PACS for medical image).

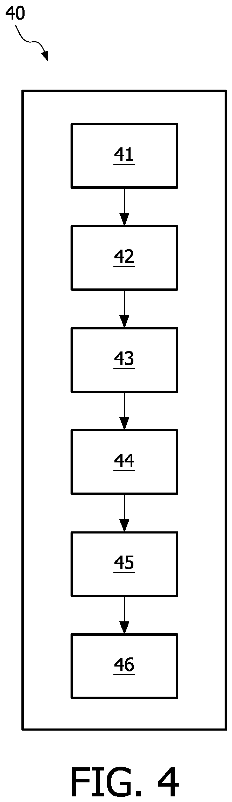

In an embodiment, according to Fig. 4, a computer-readable medium is provided having embodied thereon a computer program for processing by a processor. The computer program comprises a first code segment 41 for building 110 a DNA spectrogram database based on a DNA database comprising a number of sequences of nucleotides, by calculating an energy spectral density value for a group of nucleotides comprised in the DNA database; a second code segment 42 for inputting 120 a DNA query sequence; a third code segment 43 for calculating 130 an energy spectral density value for the DNA query sequence, resulting in an energy spectral density query; a fourth code segment 44 for calculating a difference 140 between the energy spectral density query value and an energy spectral density value comprised in the DNA spectrogram database; and a fifth code segment 45 for selecting 150 a difference being lower than a predetermined threshold value.

The computer program further comprise a sixth code segment for performing 46 sequence alignment the nucleotides comprised in a selected group.

In an embodiment the computer program comprises code segments arranged, when run by an apparatus having computer-processing properties, for performing any one of the method steps defined in some embodiments.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. The terms DNA sequence and DNA spectrogram database, as represented in the claims, may be any nucleotide sequence, or nucleotide spectrogram database, which is easily understood by a person skilled in the art. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

**Claims**

1. A computer-implemented method (10) for DNA sequence alignment of sequences with large number of nucleotides, comprising:

    building (110) a DNA spectrogram database based on a DNA database comprising a number of sequences of nucleotides, by calculating an energy spectral density value for nucleotides comprised in said DNA database, inputting (120) a DNA query sequence;
    calculating (130) an energy spectral density value for said DNA query sequence, resulting in an energy spectral density query;
    calculating a difference (140) between said energy spectral density query value and an energy spectral density value comprised in the DNA spectrogram database;
    selecting (150) a calculated difference, pertaining to a first group of nucleotides, being within a predetermined threshold value range ($\pm \Phi_\Delta$); and performing sequence alignment (160) on said first group of nucleotides from the DNA spectrogram database.

2. The method according to claim 1, wherein said DNA spectrogram database is a genomic energy spectral density database.

3. The method according to claim 3, wherein said sequence alignment (160) is local alignment.

4. The method according to claim 3, wherein said sequence alignment (160) is global alignment.

5. A device comprising a processor unit configured to:

    build (31) a DNA spectrogram database based on a DNA database comprising a number of sequences of nucleotides, by calculating an energy spectral density value for nucleotides comprised in the DNA database;
    receive (32) a DNA query sequence;
    calculate (33) an energy spectral density value for the DNA query sequence, resulting in an energy spectral density query;
    calculate (34) a difference between the energy spectral density query value and an energy spectral density value comprised in the DNA spectrogram database; select (35) a difference being lower than a predetermined threshold value; and
    perform (36) sequence alignment of the nucleotides comprised in a selected group.

6. A computer-readable medium having embodied thereon a computer program for processing by a processor, said computer program comprising:

    a first code segment (41) for building a DNA spectrogram database based on a DNA database comprising a number of sequences of nucleotides, by calculating an energy spectral density value for nucleotides comprised in the DNA database;
    a second code segment (42) for inputting a DNA query sequence;
    a third code segment (43) for calculating an energy spectral density value for the DNA query sequence, resulting in an energy spectral density query;
    a fourth code segment (44) for calculating a difference between the energy spectral density query value and an energy spectral density value comprised in the DNA spectrogram database;
    a fifth code segment (45) for selecting a difference being lower than a predetermined threshold value; and
    a sixth code segment (46) for performing sequence alignment of the nucleotides comprised in a selected group.

**Patentansprüche**

1. Computerimplementiertes Verfahren (10) zur DNA-Sequenzausrichtung von Sequenzen mit einer großen Anzahl von Nukleotiden, umfassend:

    Aufbauen (110) einer DNA-Spektrogramm-Datenbank basierend auf einer DNA-Datenbank umfassend eine Anzahl von Nukleotidsequenzen durch Berechnen eines spektralen Energiedichtewerts für in der genannten DNA-Datenbank enthaltene Nukleotide,

Eingeben (120) einer DNA-Abfragesequenz;

Berechnen (130) eines spektralen Energiedichtewerts für die genannte DNA-Abfragesequenz, wodurch sich eine spektrale Energiedichteabfrage ergibt;

Berechnen einer Differenz (140) zwischen dem genannten spektralen Energiedichteabfragewert und einem in der DNA-Spektrogramm-Datenbank enthaltenen spektralen Energiedichtewert;

Auswählen (150) einer berechneten Differenz, die zu einer ersten Gruppe von Nukleotiden gehört und innerhalb eines vorgegebenen Schwellenwertbereichs ($\pm \Phi_\Delta$) liegt; und

Durchführen einer Sequenzausrichtung (160) für die genannte erste Gruppe von Nukleotiden aus der DNA-Spektrogramm-Datenbank.

2. Verfahren nach Anspruch 1, wobei die genannte DNA-Spektrogramm-Datenbank eine genomische spektrale Energiedichte-Datenbank ist.

3. Verfahren nach Anspruch 1, wobei die genannte Sequenzausrichtung (160) eine lokale Ausrichtung ist.

4. Verfahren nach Anspruch 1, wobei die genannte Sequenzausrichtung (160) eine globale Ausrichtung ist.

5. Vorrichtung umfassend eine Prozessoreinheit, die konfiguriert ist zum:

Aufbauen (31) einer DNA-Spektrogramm-Datenbank basierend auf einer DNA-Datenbank umfassend eine Anzahl von Nukleotidsequenzen durch Berechnen eines spektralen Energiedichtewerts für in der DNA-Datenbank enthaltene Nukleotide;

Empfangen (32) einer DNA-Abfragesequenz;

Berechnen (33) eines spektralen Energiedichtewerts für die DNA-Abfragesequenz, wodurch sich eine spektrale Energiedichteabfrage ergibt;

Berechnen (34) einer Differenz zwischen dem spektralen Energiedichteabfragewert und einem in der DNA-Spektrogramm-Datenbank enthaltenen spektralen Energiedichtewert;

Auswählen (35) einer Differenz, die kleiner ist als ein vorgegebener Schwellenwert; und

Durchführen (36) einer Sequenzausrichtung der in einer ausgewählten Gruppe enthaltenen Nukleotide.

6. Computerlesbares Medium, auf dem ein Computerprogramm zur Verarbeitung durch einen Prozessor verkörpert ist, wobei das genannte Computerprogramm Folgendes umfasst:

ein erstes Codesegment (41) zum Aufbauen einer DNA-Spektrogramm-Datenbank basierend auf einer DNA-Datenbank umfassend eine Anzahl von Nukleotidsequenzen durch Berechnen eines spektralen Energiedichtewerts für in der DNA-Datenbank enthaltene Nukleotide;

ein zweites Codesegment (42) zum Eingeben einer DNA-Abfragesequenz;

ein drittes Codesegment (43) zum Berechnen eines spektralen Energiedichtewerts für die DNA-Abfragesequenz, wodurch sich eine spektrale Energiedichteabfrage ergibt;

ein viertes Codesegment (44) zum Berechnen einer Differenz zwischen dem spektralen Energiedichteabfragewert und einem in der DNA-Spektrogramm-Datenbank enthaltenen spektralen Energiedichtewert;

ein fünftes Codesegment (45) zum Auswählen einer Differenz, die kleiner ist als ein vorgegebener Schwellenwert; und

ein sechstes Codesegment (46) zum Durchführen einer Sequenzausrichtung der in einer ausgewählten Gruppe enthaltenen Nukleotide.

**Revendications**

1. Procédé (10) mis en oeuvre par ordinateur pour l'alignement de séquence ADN de séquences ayant un grand nombre de nucléotides, comprenant :

la construction (110) d'une base de données de spectrogrammes d'ADN basée sur une base de données d'ADN comprenant un certain nombre de séquences de nucléotides, en calculant une valeur de densité spectrale d'énergie pour des nucléotides compris dans ladite base de données d'ADN,

l'entrée (120) d'une séquence d'interrogation d'ADN ;

le calcul (130) d'une valeur de densité spectrale d'énergie pour ladite séquence d'interrogation d'ADN, aboutissant à une interrogation de densité spectrale d'énergie ;

le calcul d'une différence (140) entre ladite valeur d'interrogation de densité spectrale d'énergie et d'une valeur de densité spectrale d'énergie comprise dans la base de données de spectrogrammes d'ADN ;
la sélection (150) d'une différence calculée, se rapportant à un premier groupe de nucléotides, étant dans les limites d'une plage prédéterminée de valeurs de seuil ($\pm\ \Phi_\Delta$) ; et
l'exécution d'un alignement de séquence (160) sur ledit premier groupe de nucléotides à partir de la base de données de spectrogrammes d'ADN.

2. Procédé selon la revendication 1, dans lequel ladite base de données de spectrogrammes d'ADN est une base de données de densités spectrales d'énergie génomique.

3. Procédé selon la revendication 1, dans lequel ledit alignement de séquence (160) est un alignement local.

4. Procédé selon la revendication 1, dans lequel ledit alignement de séquence (160) est un alignement global.

5. Dispositif comprenant une unité formant processeur configurée pour :

construire (31) une base de données de spectrogrammes d'ADN basée sur une base de données d'ADN comprenant un certain nombre de séquences de nucléotides, en calculant une valeur de densité spectrale d'énergie pour des nucléotides compris dans la base de données d'ADN ;
recevoir (32) une séquence d'interrogation d'ADN ;
calculer (33) une valeur de densité spectrale d'énergie pour la séquence d'interrogation d'ADN, aboutissant à une interrogation de densité spectrale d'énergie ;
calculer (34) une différence entre la valeur d'interrogation de densité spectrale d'énergie et une valeur de densité spectrale d'énergie comprise dans la base de données de spectrogrammes d'ADN ;
sélectionner (35) une différence qui est inférieure à une valeur de seuil prédéterminée ; et
effectuer (36) un alignement de séquence des nucléotides compris dans un groupe sélectionné.

6. Support lisible par ordinateur ayant un programme informatique incorporé en son sein pour traitement par un processeur, ledit programme informatique comprenant :

un premier segment de code (41) pour construire une base de données de spectrogrammes d'ADN basée sur une base de données d'ADN comprenant un certain nombre de séquences de nucléotides, en calculant une valeur de densité spectrale d'énergie pour des nucléotides compris dans la base de données d'ADN ;
un deuxième segment de code (42) pour entrer une séquence d'interrogation d'ADN ;
un troisième segment de code (43) pour calculer une valeur de densité spectrale d'énergie pour la séquence d'interrogation d'ADN, aboutissant à une interrogation de densité spectrale d'énergie ;
un quatrième segment de code (44) pour calculer une différence entre la valeur d'interrogation de densité spectrale d'énergie et une valeur de densité spectrale d'énergie comprise dans la base de données de spectrogrammes d'ADN ;
un cinquième segment de code (45) pour sélectionner une différence qui est inférieure à une valeur de seuil prédéterminée ; et
un sixième segment de code (46) pour effectuer un alignement de séquence des nucléotides compris dans un groupe sélectionné.

10

```
┌─────────────┐
│     110     │
└─────────────┘
       │
       ▼
┌─────────────┐
│     120     │
└─────────────┘
       │
       ▼
┌─────────────┐
│     130     │
└─────────────┘
       │
       ▼
┌─────────────┐
│     140     │
└─────────────┘
       │
       ▼
┌─────────────┐
│     150     │
└─────────────┘
       │
       ▼
┌─────────────┐
│     160     │
└─────────────┘
```

# FIG. 1

110

| 111 |

| 112 |

| 113 |

| 114 |

FIG. 2

30

```
┌─────────────────────────┐
│   ┌─────────────┐       │
│   │     31      │       │
│   └─────────────┘       │
│          │              │
│          ▼              │
│   ┌─────────────┐       │
│   │     32      │       │
│   └─────────────┘       │
│          │              │
│          ▼              │
│   ┌─────────────┐       │
│   │     33      │       │
│   └─────────────┘       │
│          │              │
│          ▼              │
│   ┌─────────────┐       │
│   │     34      │       │
│   └─────────────┘       │
│          │              │
│          ▼              │
│   ┌─────────────┐       │
│   │     35      │       │
│   └─────────────┘       │
│          │              │
│          ▼              │
│   ┌─────────────┐       │
│   │     36      │       │
│   └─────────────┘       │
└─────────────────────────┘
```

# FIG. 3

40

FIG. 4

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007105150 A